# EUROPEAN PATENT APPLICATION

(11) **EP 4 397 301 A1**
(43) Date of publication of application: **10.07.2024**
(21) Application number: 22864973.7
(22) Date of filing: 29.08.2022
(51) Int. Cl.: A61K 8/9789, A61K 8/60, A61Q 19/00, A61Q 19/08

(54) **FRUIT-DERIVED VESICLES AND COSMETIC COMPOSITION COMPRISING SAME**

(30) Priority: 01.09.2021 KR 20210116452
(71) Applicant: CG Health Tech Inc., Wonju-si, Gangwon-do 26460 (KR)
(72) Inventor: LEE, Sam yeon, Seoul 06375 (KR); YOON, Song Hee, Gwangju-si Gyeonggi-do 12798 (KR)
(74) Representative: Danubia Patent & Law Office LLC
(86) International application number: PCT/KR2022/012854
(87) International publication number: WO 2023/033471

(57) **Abstract**

The present disclosure relates to fruit-derived vesicles and a cosmetic composition including the same. More particularly, the present disclosure relates to confirming the skin soothing, skin barrier improvement, skin moisturizing, skin whitening, hair regeneration promotion, hair loss prevention, and gray hair improvement effects of various biomolecules, including miRNA and cargos, contained in the vesicles.

## Description

### BACKGROUND OF THE DISCLOSURE

### Field of the Disclosure

The present disclosure relates to fruit-derived vesicles and a cosmetic composition including the same. More particularly, the present disclosure relates to confirming the skin whitening, skin moisturizing, and skin soothing effects of various biomolecules, including miRNA and cargos, contained in the vesicles.

### Description of the Related Art

Recently, research has reported that secretomes contain various bioactive factors that control cell behavior. In particular, the secretomes include 'exosomes' or 'extracellular vesicles', which are nano-vesicles that have a signaling function between cells, and research on the components and functions of these nano-vesicles is actively in progress.

Cells release various membrane-type vesicles into the extracellular environment. Typically, these released vesicles are called extracellular vesicles (EVs). Extracellular vesicles are also called membrane-derived vesicles, ectosomes, shedding vesicles, microparticles, and exosomes.

Exosomes are vesicles that are made of a double phospholipid membrane similar to the structure of a cell membrane and have a size of tens to hundreds of nanometers, and exosomes contain proteins called exosome cargos, mRNA, and miRNA. Exosome cargos include a wide range of signaling factors, and these signaling factors are known to be cell type specific and differently regulated depending on the secretory cell environment. Exosomes are intercellular signaling mediators secreted by cells, and various cell signals transmitted through exosomes are known to regulate cell behavior, including activation, growth, migration, differentiation, dedifferentiation, apoptosis, and necrosis of target cells. Depending on the nature and state of the cell from which exosomes are derived, the exosomes contain specific genetic materials and bioactive factors. In the case of proliferating stem cell-derived exosomes, the exosomes regulate cell behavior such as cell migration, proliferation, and differentiation and have stem cell characteristics related to tissue regeneration (Nature Review Immunology 2002 (2) 569-579).

Most animal cells have the ability to secrete cell-derived extracellular vesicles of various sizes and compositions. These extracellular vesicles are found in all biological fluids, including blood, urine, saliva, and cell cultures (Loyer X, Vion AC, Tedgui A, Boulanger CM. Microvesicles as cell-cell messengers in cardiovascular diseases. Circ Res 2014;114:345-53)(Ohno S, Ishikawa A, Kuroda M. Roles of exosomes and microvesicles in disease pathogenesis. Adv Drug Deliv Rev 2013;65:398-401).

Extracellular vesicles reflect the state of the origin (donor) cell that secretes the extracellular vesicles. Depending on the type of secretory cell, extracellular vesicles exhibit various biological activities and play an important role in cell-cell interaction by transferring genetic materials and proteins between cells.

Also in plants, fusion of the plasma membrane with the multivesicular body results in the release of small vesicles into the extracellular space. In various plant cells of various plant species, multivesicular internal vesicles are observed in the extracellular space (Marchant R, Peat A, Banbury GH. The ultrastructural basis of hyphal growth. New Phytol. 1967;66:623-629)(Halperin W, Jensen WA. Ultrastructural changes during growth and embryogenesis in carrot cell cultures. J Ultrastruct Res. 1967;18:428-443)(Marchant R, Robards AW. Membrane systems associated with the plasmalemma of plant cells. Ann Bot. 1968;32:457-471).

In addition, in a recent study, exosome-like nanoparticles derived from plant cells were reported to have similar nano-sized vesicle structure and nanoparticle composition to exosomes derived from mammalian cells (An, Q, Huckelhoven, R, Kogel, KH and van Bel, AJ (2006). Multivesicular bodies participate in a cell wall-associated defence response in barley leaves attacked by the pathogenic powdery mildew fungus. Cell Microbiol 8: 1009-1019)(Regente, M, Pinedo, M, Elizalde, M and de la Canal, L (2012). Apoplastic exosomelike vesicles: a new way of protein secretion in plants? Plant Signal Behav 7: 544-546).

Conventionally, exosomes have been mainly used as biomarkers, and technology to use exosomes for specific purposes by utilizing the efficacy of the exosomes is still underdeveloped.

In particular, vesicles derived from plant cells have no known use. In particular, there have been no reports on vesicles obtained from fruits and cosmetic compositions using the vesicles.

### SUMMARY OF THE DISCLOSURE

Therefore, the present disclosure has been made in view of the above problems, and it is an object of the present disclosure to provide a composition that is effective in improving skin by specifying and analyzing fruit-derived vesicles and various biomolecules (cargoes) including miRNA contained in the vesicles.

It is another object of the present disclosure to provide a cosmetic composition including the fruit-derived vesicles.

It is yet another object of the present disclosure to provide a cosmetic composition that exhibits complex effects such as skin soothing, skin barrier strengthening, skin moisturizing, and skin whitening at the miRNA level and a method of preparing the cosmetic composition.

In accordance with one aspect of the present disclosure, provided is a cell-free composition including biomolecules extracted from a fruit together with at least one carrier, excipient or diluent.

In the present disclosure, the biomolecules may include one or more selected from proteins, peptides, antigens, antibodies, protein fragments, DNA, RNA, cells, microvesicles, and other bioparticles, preferably extracellular vesicles and miRNA, without being limited thereto.

In the present disclosure, the carrier, excipient, and diluent may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

The composition of the present disclosure is characterized by the absence of cells, which is advantageous in this respect because intravenous administration of cells may cause embolism, blood aggregation, and immune response.

Specifically, the biomolecules may be vesicles obtained directly from a fruit, and the biomolecules may be miRNA contained in fruit-derived vesicles and fruit extract-derived vesicles.

The fruit may include one or more selected from the group consisting of tangerine (*Citrus reticulata)* belonging to the genus *Citrus,* orange (*Citrus sinensis*), lemon (*Citrus limon),* citron (*Citrus junos)*, lime (*Citrus latifolia)*, grapefruit (*Citrus sinensis*), kumquat (*Citrus japonica*), pear (*Pyruspyrifolia,* Korean pear), oriental melon (*Cucumis melo* ssp. *agrestis* var. *makuwa*), specifically tangerine belonging to the genus *Citrus,* without being limited thereto.

Specifically, the biomolecules may be miRNAs commonly expressed in tangerine, and may include one or more selected from the group consisting of aly-mir159a-3p, aly-mir164a-5p, cme-mir399b, atr-mir166b, aly-mir396a-5p, bdi-mir398a, ppe-mir827, aly-mir168a-3p, ath-mir827, aly-mir396a-3p, aly-mir403a-3p, cme-mir399b, bdi-mir398a, ppe-mir827, ath-mir827, aly-mir167d-5p, ppe-mir858, atr-mir319b, aly-mir170-5p, gma-mir393h, gma-mir390e, gma-mir171b-3p, tcc-mir399e, gma-mir390a-3p, aly-mir167b-3p, ata-mir395a-3p, mtr-mir319c-3p, and aly-mir390a-3p, more specifically, one or more selected from the group consisting of aly-mir159a-3p, aly-mir164a-5p, cme-mir399b, atr-mir166b, aly-mir396a-5p, bdi-mir398a, ppe-mir827, aly-mir168a-3p, ath-mir827, aly-mir396a-3p, and aly-mir403a-3p, which are top 11 miRNAs in terms of expression level, most specifically, aly-mir159a-3p, aly-mir164a-5p, and cme-mir399b, which are top three miRNAs in terms of expression level.

MicroRNA (miRNA) refers to small non-coding RNA consisting of 18 to 25 nucleotides in base sequence length.

In an embodiment of the present disclosure, it was confirmed that all of the top three miRNAs in terms of expression level have effects related to skin health and whitening.

In accordance with another aspect of the present disclosure, provided is a cosmetic composition including the cell-free composition.

The cosmetic composition is characterized by having skin soothing, skin barrier improvement, skin moisturizing, and skin whitening effects separately or simultaneously. In addition, the cosmetic composition may have hair regeneration promotion, hair loss prevention, and gray hair improvement effects. The effects of each of these were confirmed in Experimental Examples 1 to 6, and in particular, hair regeneration, hair loss prevention, and gray hair improvement effects were confirmed through Experimental Example 6 using hair follicle stem cells.

In the present disclosure, the term "skin barrier improvement" refers to improving the barrier function of the stratum corneum located in the outermost layer of the skin. The stratum corneum, which is the first defense layer of the skin, may be damaged by physical stimulation or wounds. It is known that in people with atopic dermatitis or dry skin, the skin barrier function of the stratum corneum is not functioning properly or is damaged. It is common to apply moisturizers to treat or protect atopic dermatitis or dry skin, but regular moisturizers have the limitation that the moisturizers simply function to replenish moisture or cover the skin and may not solve the fundamental problem.

The cosmetic composition of the present disclosure may be prepared in a formulation selected from the group consisting of solution, external ointment, cream, foam, nourishing lotion, softening lotion, pack, softening water, emulsion, makeup base, essence, soap, liquid cleanser, bath preparation, sunscreen cream, sun oil, suspension, emulsion, paste, gel, lotion, powder, soap, surfactant-containing cleanser, oil, powder foundation, emulsion foundation, wax foundation, patch, and spray, without being limited thereto.

The cosmetic composition of the present disclosure may additionally include one or more cosmetically acceptable carriers mixed in general skin cosmetics, and common ingredients, for example, oil, water, a surfactant, a moisturizer, a lower alcohol, a thickener, a chelating agent, a colorant, a preservative, fragrance, etc., may be appropriately mixed, without being limited thereto.

The cosmetologically acceptable carriers included in the cosmetic composition of the present disclosure vary depending on the formulation of the cosmetic composition.

When the formulation of the present disclosure is ointment, paste, cream, or gel, as the carrier ingredients, animal oil, vegetable oil, wax, paraffin, starch, tracant, cellulose derivatives, polyethylene glycol, silicon, bentonite, silica, talc, zinc oxide, and the like may be used, without being limited thereto. The carrier ingredients may be used alone or in combination of two or more types.

When the formulation of the present disclosure is powder or spray, as the carrier ingredients, lactose, talc, silica, aluminum hydroxide, calcium silicate, polyimide powder, and the like may be used. In particular, when the formulation of the present disclosure is spray, additionally, a propellant such as chlorofluorohydrocarbon, propane/butane, or dimethyl ether may be included, without being limited thereto. The carrier ingredients may be used alone or in combination of two or more types.

When the formulation of the present disclosure is a solution or emulsion, as the carrier ingredients, solvents, solubilizers, or emulsifying agents may be used. For example, as the carrier ingredients, water, ethanol, isopropanol, ethyl carbonate, ethyl acetate, benzyl alcohol, benzyl benzoate, propylene glycol, 1,3-butyl glycol oil, and the like may be used. In particular, as the carrier ingredients, cottonseed oil, peanut oil, corn germ oil, olive oil, castor oil, sesame oil, glycerol aliphatic ester, polyethylene glycol, or fatty acid esters of sorbitan may be used, without being limited thereto. The carrier ingredients may be used alone or in combination of two or more types.

When the formulation of the present disclosure is a suspension, as the carrier ingredients, liquid diluents such as water, ethanol, or propylene glycol, suspending agents such as ethoxylated isostearyl alcohol, polyoxyethylene sorbitol ester, and polyoxyethylene sorbitan ester, microcrystalline cellulose, aluminum metahydroxide, bentonite, agar, or tracant may be used, without being limited thereto. The carrier ingredients may be used alone or in combination of two or more types.

Specifically, in the present disclosure, one or more selected from the group consisting of lactic acid, capryloyl salicylic acid, lactobionic acid, hydrogenated lecithin, xylityl glucoside, anhydroxylitol, stearic acid, glucose, ceramide NP, phytosphingosine, ceramide NS, cholesterol, ethylhexylglycerin, ceramide AS, ceramide AP, ceramide EOP, ethylhexyl palmitate, pentylene glycol, caprylic/capric triglyceride, propylene glycol, glycolic acid, 1,2-hexanediol, ceteth-20, glyceryl stearate, sorbitan stearate, cetearyl alcohol, methyl glucose sesquistearate, cyclodextrin, polyglyceryl-4-isostearate, sodium hydroxide, sclerotium gum, glycerin, polyglyceryl-4-caprate, and butylene glycol may be included, without being limited thereto.

### BRIEF DESCRIPTION OF THE DRAWINGS

The above and other objects, features and other advantages of the present disclosure will be more clearly understood from the following detailed description taken in conjunction with the accompanying drawings, in which:

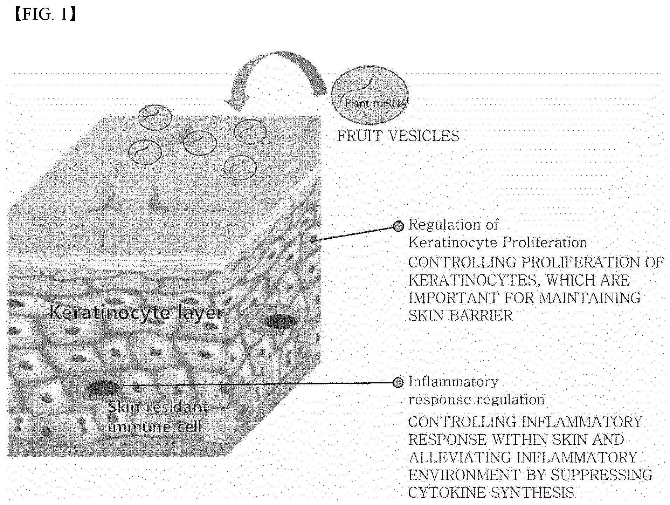

FIG. 1 shows the effects of fruit-derived vesicles and miRNAs contained in the fruit-derived vesicles on the skin, showing effects such as controlling keratinocyte proliferation, suppressing cytokine synthesis, and inhibiting melanin biosynthesis at the molecular level;
FIG 2 shows the results of evaluating cell viability when cells are treated with pear-, tangerine-, and oriental melon-derived vesicles of the present disclosure;
FIG. 3 shows the results of evaluating melanin biosynthesis inhibition efficacy when cells are treated with the pear-, tangerine-, and oriental melon-derived vesicles of the present disclosure;
FIG. 4 shows the results of evaluating skin regeneration effects when cells are treated with the pear-, tangerine-, and oriental melon-derived vesicles of the present disclosure;
FIG. 5 shows the results of evaluating antioxidative effects when cells are treated with the pear-, tangerine-, and oriental melon-derived vesicles of the present disclosure;
FIG. 6 shows the results of evaluating cell viability when cells are treated with the pear-, tangerine-, and oriental melon-derived miRNAs of the present disclosure;
FIG. 7 shows the results of evaluating melanin biosynthesis inhibition efficacy when cells are treated with the pear-, tangerine-, and oriental melon-derived miRNAs of the present disclosure;
FIG. 8 shows the results of evaluating skin regeneration effects when cell are treated with the pear-, tangerine-, and oriental melon-derived miRNAs of the present disclosure;
FIG. 9 shows the results of evaluating antioxidative effects when cells are treated with the pear-, tangerine-, and oriental melon-derived miRNAs of the present disclosure;
FIG. 10 shows the results of evaluating anti-inflammatory effects by using real-time PCR when the BV2 cell line is treated with the pear-, tangerine-, and oriental melon-derived vesicles of the present disclosure;
FIG. 11 shows the results of evaluating anti-inflammatory effects by using real-time PCR when the BV2 cell line is treated with the pear-, tangerine-, and oriental melon-derived miRNAs of the present disclosure and then treated with 1 µg/mL of LPS;
FIG. 12 shows the results of evaluating cell proliferation ability when a hair follicle stem cell line is treated with the pear-, tangerine-, and oriental melon-derived miRNAs of the present disclosure and then treated with 100 µM H₂O₂; and
FIG. 13 shows the results of evaluating antioxidative effects by using DPPH-assay when a hair follicle stem cell line is treated with the pear-, tangerine-, and oriental melon-derived miRNAs of the present disclosure (Positive Control: ascorbic acid (A.A) 50, 100 µM).

### DETAILED DESCRIPTION OF THE DISCLOSURE

Hereinafter, the present disclosure is described in detail by examples. However, the following examples only illustrate the present disclosure, and the present disclosure is not limited by the following examples.

### Example 1. Obtaining fruit-derived vesicles

After preparing pear, tangerine, and oriental melon, each fruit was finely chopped, filtered through gauze, and then centrifuged at 1,000 to 2,000 g for 10 to 60 minutes. The supernatant was filtered through a 40 µm mesh and centrifuged at 2,000 to 3,000 g for 30 to 60 minutes. Then, the supernatant was collected and filtered once more through a 0.8 to 1.5 µm mesh.

The filtered supernatant was centrifuged at 10,000 g to 20,000 g for 1 to 3 hours and centrifuged at 100,000 to 150,000 g for 1 to 2 hours to remove the supernatant. Then, fruit-derived vesicles were obtained by suspending pelleted extracellular vesicles with DPBS.

### Experimental Example 1. Cytotoxicity assay

To confirm the cytotoxicity of the fruit-derived vesicles of the present disclosure, B 16-F10 (ATCC CRL-6475) was treated with the fruit-derived vesicles of Example 1 and the mixture thereof, and then a cytotoxicity assay was performed.

Specifically, the cytotoxicity effect of the vesicles was evaluated by MTT assay (3-(4,5-dimethylthiazol-2-yl)-2,5-diphenyltetrazolium bromide assay).

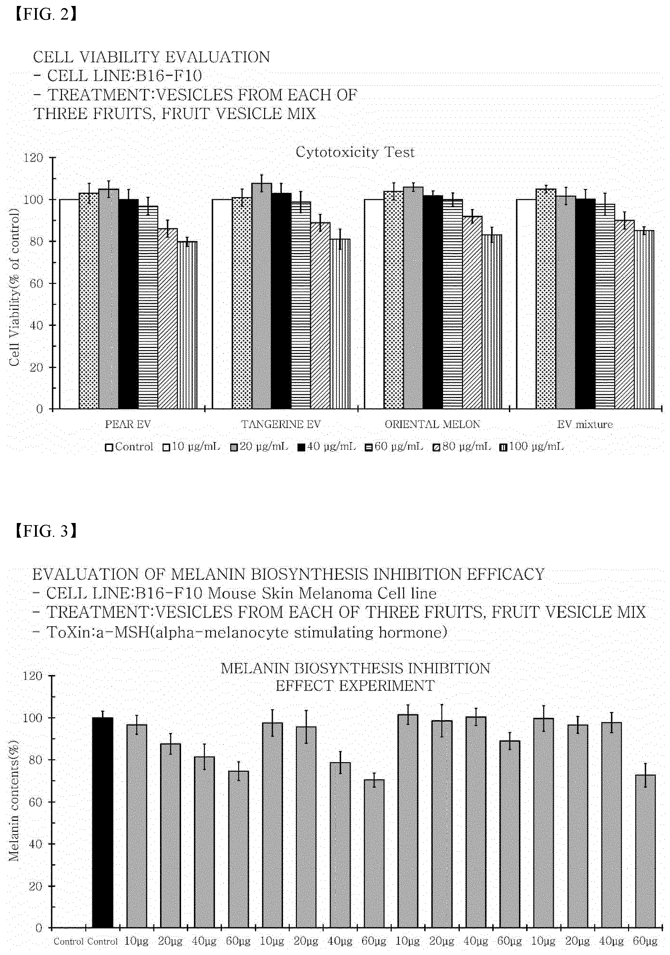

As a result, as shown in FIG. 2, the fruit-derived vesicles of the present disclosure were confirmed not to exhibit cytotoxicity.

In addition, after B16-F10 cells were treated under the same conditions with the three most abundant (highest expression) miRNAs in the fruit-derived vesicles of the present disclosure, cytotoxicity was confirmed.

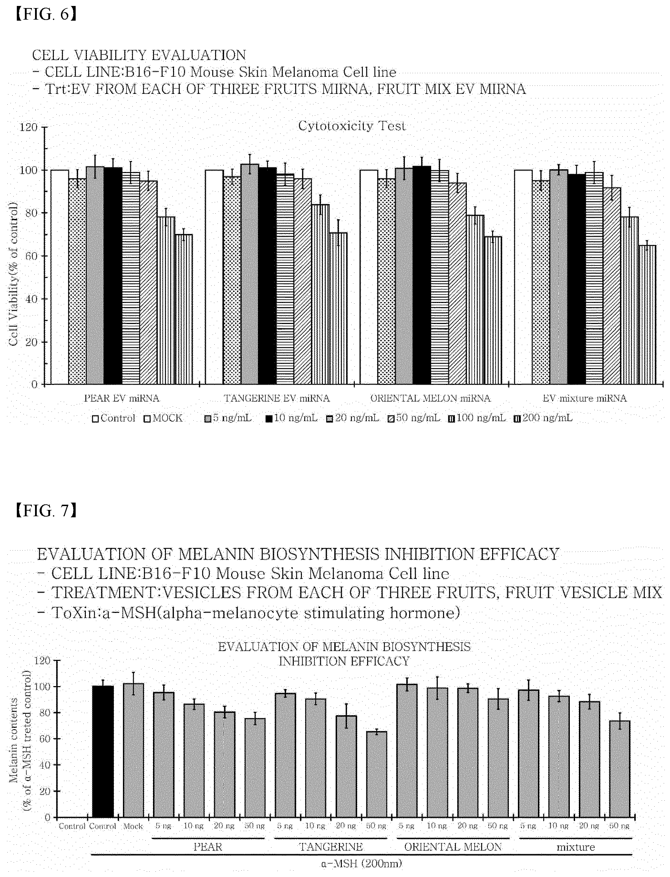

As a result, as shown in FIG. 6, it was confirmed that even when treated with miRNAs at a concentration of about 50 ng/mL, the cell survival rate exceeded 90 % and cytotoxicity was not observed.

### Experimental Example 2. Evaluation of melanin biosynthesis inhibition efficacy

An experiment was conducted to determine whether treatment with the fruit-derived vesicles of the present disclosure had a whitening effect by inhibiting the biosynthesis of melanin.

Specifically, to measure melanin production in B16-F10 cells, the cells were aliquoted to each well of a 24-well plate at 2.0 × 10 ⁴ cells/well and incubated for 24 hours in a 5 % CO₂ incubator set to 37 °C. Then, to induce melanin biosynthesis, the culture medium of the cells was replaced with culture medium containing 200 nM α-MSH, and then the fruit-derived vesicles and miRNAs of Example 1 were added thereto according to concentrations, followed by incubation for 24 hours in a 5 % CO₂ incubator set to 37 °C.

Then, the culture medium was removed, the 24 wells were washed with 0.5 mL of PBS, and the cells were treated with trypsin-EDTA to recover the cells. 200 µL of 1 N NaOH was added to the recovered cells, and the cells were placed in a water bath at 80 °C for 1 hour to dissolve melanin within the cells. Then, the cells were placed on ice for 1 hour. Melanin content was measured at 405 nm using ELISA.

As a result, as shown in FIGS. 3 and 7, when treated with the fruit-derived vesicles or miRNAs of the present disclosure, a significant biosynthetic inhibition effect was observed. In particular, pear and tangerine showed notable effects.

### Experimental Example 3. Evaluation of skin regeneration effect

To determine whether treatment with the fruit-derived vesicles of the present disclosure promotes skin regeneration, the expression of factors related to skin regeneration was analyzed after treatment with the fruit-derived vesicles.

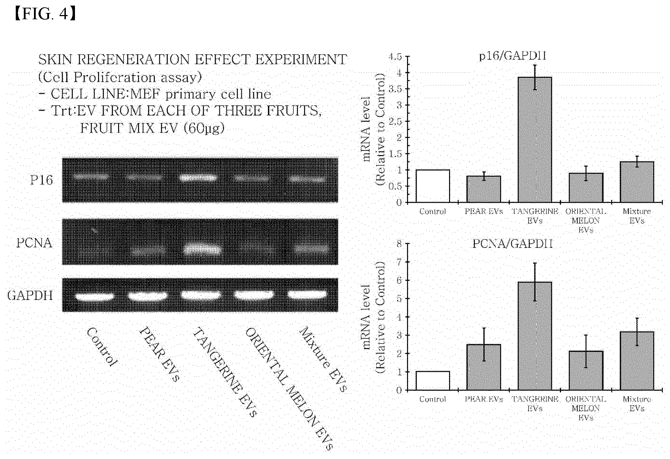

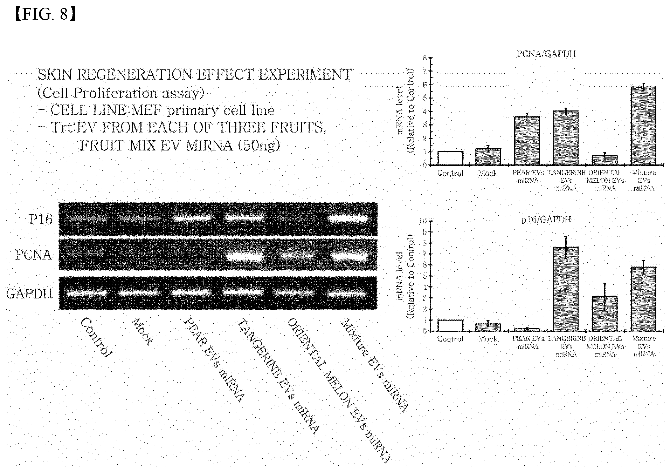

Specifically, the expression levels of *p16* and *PCNA* (Proliferating Cell Nuclear Antigen), which are factors related to the cell cycle, were compared with the expression of GAPDH. The results obtained after treatment with the fruit-derived vesicles are shown in FIG. 4, and the results obtained after treatment with miRNAs are shown in FIG. 8.

According to the experimental method, cell samples were obtained from a group (transfection) in which mouse embryonic fibrocytes were treated with miRNAs isolated from the fruit-derived vesicles, and RNA was extracted from each sample.

After dissolving 2 µg of the extracted RNA in DEPC-treated water, 1 µL of oligo(dt) 12-18 and 1 µL of 10 mM dNTP were added thereto. Then, the mixture was reacted at 70 °C for 5 minutes, and immediately cooled on ice. Then, 4 µL of 5 × reaction (first strand) buffer, 2 µL of 100 mM DTT, and 40 unit of an RNase inhibitor were added to the mixture, and the mixture was reacted at 42 °C for 50 minutes and reacted at 65 °C for 10 minutes to synthesize cDNA.

1 µL of the synthesized cDNA, 5 µL of 10 × reaction buffer, 1 µL of each of 2 types of cell proliferation-related gene primer pairs (50 pmole), 5 µL of 10 mM dNTP, 0.5 µL of Taq polymerase, and an appropriate amount (up to 50 µL) of distilled water were mixed. Then, the mixture was heated at 94 °C for 5 minutes, one cycle of 95 °C for 30 seconds, 55 °C for 30 seconds, and 72 °C for 30 seconds was repeated 30 times, and then the reaction was terminated by heating at 72 °C for 10 minutes. The obtained sample was confirmed through 1.5 % agarose gel.

As a result, when treated with vesicles or miRNAs derived from tangerine, the expression of genes related to skin regeneration was significantly increased.

In particular, it was confirmed that when tangerine-derived vesicles or miRNAs were mixed with vesicles or miRNAs derived from other fruits, the effect was significantly reduced.

### Experimental Example 4. Evaluation of antioxidative effect

To determine whether treatment with the fruit-derived vesicles of the present disclosure affects the antioxidative effect, DCF-DA assay was performed.

DCF-DA assay is a test method that evaluates the level of generated reactive oxygen species based on the concentration of DCF generated when DCF-DA reacts with reactive oxygen species within cells. Specifically, mouse fibroblasts were treated with each of fruit-derived vesicles (60 µg) or fruit-extracted miRNAs (50 ng) for 24 hours, and then treated with 100 ng/mL TNF-a for 1 hour. Then, it was confirmed whether the fruit vesicles and the miRNAs inhibit the production of reactive oxygen species in stimulated cells.

The medium was removed from the treated cells, the cells were washed with PBS, 20 µM DCF-DA was added to the cells, and the cells were then incubated for 30 minutes. Then, the cells were washed twice with PBS, and fluorescence was observed at an excitation wavelength of 485 nm and an emission wavelength of 535 nm using a fluorescence microscope. Then, each fluorescence intensity was measured and quantitatively compared through a graph.

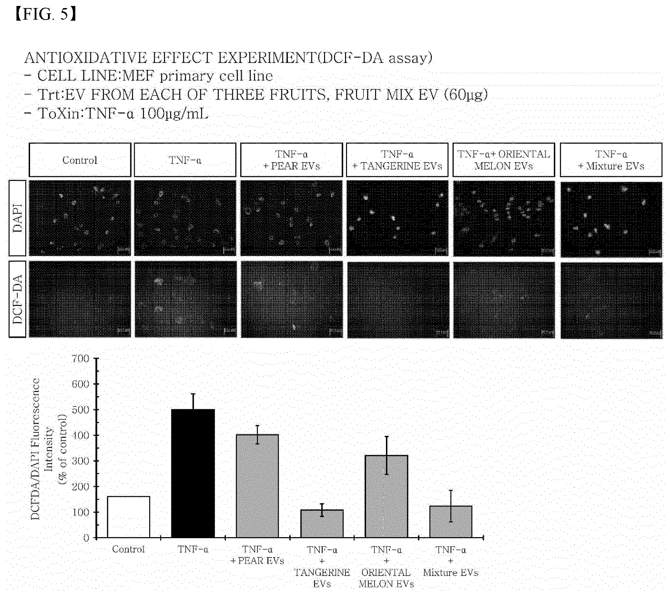

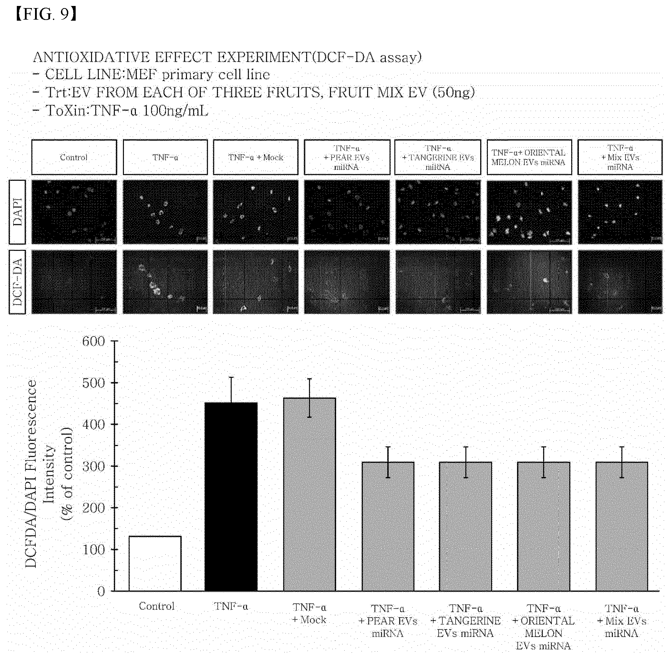

The results of treatment with the fruit-derived vesicles are shown in FIG. 5, and the results of treatment with miRNAs are shown in FIG. 9.

As a result, as shown in FIG. 5, when treated with the tangerine-derived vesicles, the results were similar to the control group, confirming a significantly superior antioxidative effect. The case of FIG. 9 treated with miRNAs showed some differences from the case treated with the vesicles, but it was confirmed that the case of FIG. 9 showed a significantly superior antioxidative effect.

### Experimental Example 5. Evaluation of anti-inflammatory effect

Since an anti-inflammatory effect is expected at the molecular level when treated with the fruit-derived vesicles of the present disclosure, it was expected that the fruit-derived vesicles could be applied to skin soothing, inflammation treatment, and acne treatment. Thus, the anti-inflammatory effect was evaluated.

Specifically, the expression levels of inflammation-related factors IL-4, IL-6, IL-10, and TNF-α were confirmed.

Specifically, cell samples were obtained from a group in which the BV2 immune cell line was treated with LPS, which induces inflammation, and a group treated with miRNAs isolated from each fruit-derived vesicle (transfection), and RNA was extracted from each sample.

5 µg of the extracted RNA was dissolved in DEPC-treated water, 1 µL of oligo(dt) 12-18 and 1 µL of 10 mM dNTP were added thereto, and the mixture was reacted at 70 °C for 10 minutes, followed by cooling on ice. Then, 4 µL of 5 × first strand buffer, 2 µL of 100 mM DTT, and 40 unit of RNase OUT were added thereto, and the mixture was reacted at 42 °C for 50 minutes, followed by incubation at 65 °C for 10 minutes to synthesize cDNA.

5 µL of the synthesized cDNA, 1 µL of each of 4 types of inflammation-related gene primer pairs, 12.5 µL of SYBR, and an appropriate amount (up to 25 µL) of distilled water were mixed. Then, the mixture was reacted at 94 °C for 5 minutes, and one cycle of 95 °C for 1 minute and 55 °C for 30 seconds was repeated 40 times. At each cycle, the reaction was checked and recorded in real time. After the repeat cycle was completed, the mixture was heated at 72 °C for 10 minutes to terminate the reaction.

In the above experiment, the cycle recorded as causing a certain amount of reaction was calculated in proportion to the normal group and used as the result.

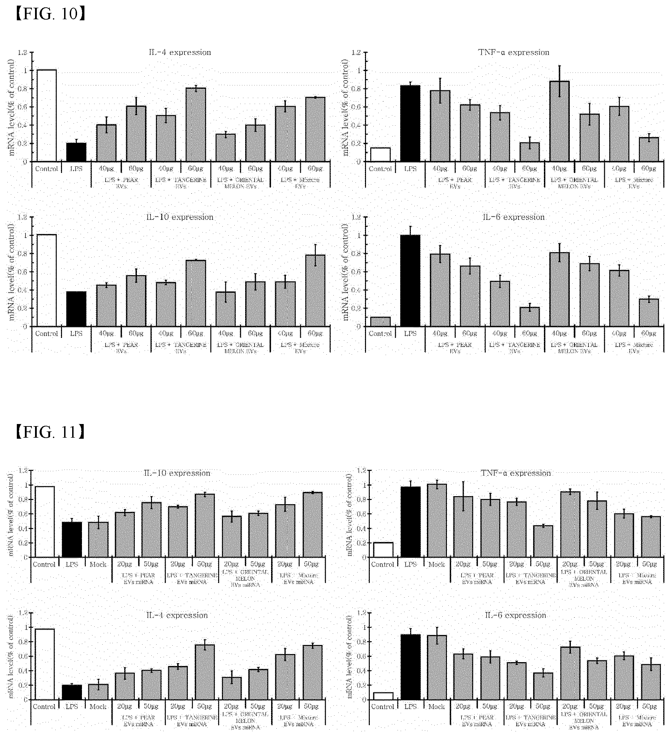

As a result, in FIG. 10 treated with the vesicles, it was confirmed that the tangerine-derived vesicles showed an effect most similar to the control group that was not treated with LPS for all four factors, showing an excellent anti-inflammatory effect.

On the other hand, FIG. 11 treated with miRNA did not show a significantly superior effect in all cases as in the case treated with the vesicles, but it was confirmed that the case of FIG. 11 showed the best effect overall.

### Experimental Example 6. Evaluation of effect when treated on hair follicle cell line

Based on the excellent antioxidant and cell regeneration effects of the fruit-derived vesicles of the present disclosure and the miRNA derived from the vesicles confirmed through the experimental examples, the hair regeneration, hair loss prevention, and gray hair improvement effects of the fruit-derived vesicles of the present disclosure and the miRNAs were confirmed.

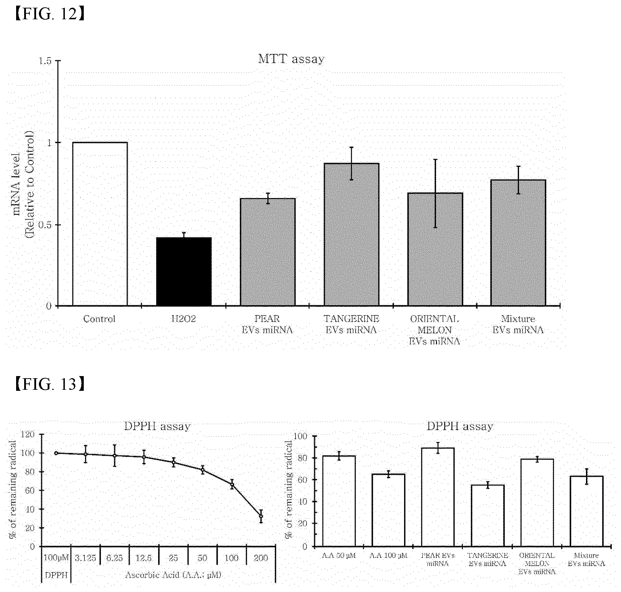

Specifically, hair follicle stem cells were treated with 100 µM H₂O₂, and then treated with miRNAs obtained from each of three fruits and mixtures thereof. Then, cell proliferation ability was confirmed through MTT-assay (FIG. 12).

In addition, the hair follicle stem cells were treated with miRNAs obtained from each of three fruits and mixtures thereof, and then DPPH-assay was performed to confirm free radical scavenging ability (FIG. 13).

As a result, all fruit-derived miRNAs of the present disclosure showed significant effects compared to the control group, and it was confirmed that tangerine had a particularly large effect.

From the above results, it was confirmed that the fruit-derived vesicles of the present disclosure and the miRNAs included in the vesicles exhibited excellent skin soothing, skin barrier improvement, skin moisturizing, and skin whitening effects simultaneously. In addition, it was confirmed that the vesicles and miRNAs of the present disclosure may help anti-oxidation of hair follicle stem cells, and thus hair regeneration, hair loss prevention, and gray hair improvement effects may be expected. In addition, the results of the experiment show that the vesicles and the bioactive substances inside the vesicles are accurately delivered to the desired target, resulting in a fundamental skin improvement effect.

In addition, the fruit-derived vesicles and the miRNAs are expected to show excellent effects when used in the form of a cosmetic composition containing the vesicles and miRNAs as the vesicles and miRNAs take the form of vesicles, which are intercellular transport substances.

The cosmetic composition including the fruit-derived vesicles of the present disclosure can exhibit excellent skin soothing, skin barrier improvement, skin moisturizing, and skin whitening effects.

In addition, the composition of the present disclosure is a cell-free composition, which is expected to minimize side effects on the human body. Furthermore, since the composition is a fruit-derived material, the composition is non-toxic, making the composition highly usable as a cosmetic composition.

The effects of the present disclosure are not limited to the above-described effects, and should be understood to include all effects that can be inferred from the configuration of the invention described in the detailed description or claims of the present disclosure.

The aforementioned description of the present disclosure is provided by way of example and those skilled in the art will understand that the present disclosure can be easily changed or modified into other specified forms without change or modification of the technical spirit or essential characteristics of the present disclosure. Therefore, it should be understood that the aforementioned examples are only provided by way of example and not provided to limit the present disclosure. For example, each of constituents described as a single form may be separately implemented and, similarly, constituents described as being separated may be implemented in a combined form.

It should be understood that the scope of the present disclosure is defined by the following claims and the invention is to cover all modifications, equivalents, and alternatives falling within the spirit and scope of the invention as defined by the claims.

## Claims

1. A cell-free composition, comprising biomolecules extracted from a fruit together with at least one carrier, excipient or diluent.

2. The cell-free composition according to claim 1, wherein the biomolecules are vesicles obtained from a fruit.

3. The cell-free composition according to claim 2, wherein the biomolecules are miRNAs comprised in the vesicles.

4. The cell-free composition according to claim 3, wherein the miRNA comprises one or more selected from the group consisting of aly-mir159a-3p, aly-mir164a-5p, cme-mir399b, atr-mir166b, aly-mir396a-5p, bdi-mir398a, ppe-mir827, aly-mir168a-3p, ath-mir827, aly-mir396a-3p, and aly-mir403a-3p.

5. A cosmetic composition, comprising the cell-free composition according to claim 1.

6. The cosmetic composition according to claim 5, wherein the cosmetic composition exhibits one or more effects selected from the group consisting of skin soothing, skin barrier improvement, skin moisturizing, skin whitening, hair regeneration promotion, hair loss prevention, and gray hair improvement.

7. The cosmetic composition according to claim 5, wherein the cosmetic composition is used for anti-inflammatory purposes.

8. The cosmetic composition according to claim 5, wherein the cosmetic composition is used for antioxidant purposes.

9. The cosmetic composition according to claim 5, wherein the cosmetic composition is used for anti-aging purposes.
